# EUROPÄISCHE PATENTSCHRIFT

(11) **EP 4 440 522 B1**
(45) Veröffentlichungstag und Bekanntmachung des Hinweises auf die Patenterteilung: **04.03.2026**
(21) Anmeldenummer: 23701938.5
(22) Anmeldetag: 24.01.2023
(51) Int. Cl.: A61F 13/0203, A61F 13/0206, A61F 13/01

(54) **WUNDAUFLAGE MIT BOGENFÖRMIGEN EINSCHNITTEN**
WOUND DRESSING HAVING ARCUATE INDENTATIONS
PANSEMENT AYANT DES INDENTATIONS ARQUÉES

(30) Priorität: 24.01.2022 DE 202022100372 U
(43) Veröffentlichungstag der Anmeldung: 09.10.2024
(73) Patentinhaber: Lohmann & Rauscher GmbH & Co. KG, 56567 Neuwied (DE)
(72) Erfinder: SCHULZE, Daniel, 53721 Siegburg (DE)
(74) Vertreter: Herrmann, Daniel
(86) Internationale Anmeldenummer: PCT/EP2023/051666
(87) Internationale Veröffentlichungsnummer: WO 2023/139283

(56) Entgegenhaltungen:
- EP-A1- 3 085 344
- CN-U- 213 465 619
- DE-A1- 102011 002 268
- GB-A- 941 591
- US-A1- 2014 094 730

## Beschreibung

Die Erfindung betrifft eine Wundauflage mit bogenförmigen Einschnitten, einen Wundverband, ein Wundversorgungskit und Verwendungen der Wundauflage, des Wundverbandes sowie des Wundversorgungskits.

Moderne Wundauflagen werden üblicherweise aus einer Vielzahl von Materialien hergestellt, um eine mehrschichtige Wundauflage zu bilden, welche ein verbessertes Exsudatmanagement ermöglicht. Zu diesen Materialien gehören Folien, Klebstoffe, Schäume und Faservlies. Die Anpassungsfähigkeit einer Wundauflage an die verwundete Körperstelle wird im Allgemeinen durch die mechanischen Eigenschaften ihrer Bestandteile bestimmt. Einige der bekannten Wundauflagen enthalten eine Vliesstoffschicht, um die Absorptionseigenschaften der Wundauflage und eine Retention von Exsudat zu verbessern. Ein Nachteil von Vliesstoff ist jedoch, dass er eine eingeschränkte Dehnbarkeit aufweist.

Eine verminderte Dehnbarkeit von Wundauflagen kann Probleme bei der Anwendung verursachen, insbesondere wenn die Wundauflagen an Stellen angebracht werden, an denen hohe Scherspannungen auf die Wundauflagen einwirken, z. B. durch Beugen eines Knies oder eines Ellbogens. Eine eingeschränkte Dehnbarkeit von Wundauflagen kann beispielsweise dazu führen, dass die Wundauflagen einen schlechten Tragekomfort aufweisen, sich die Wundauflagen teilweise von der Wunde ablösen, die Funktionalität der Wundauflagen durch eine mangelnde Wundabdeckung vermindert ist, und/oder durch eine mechanische Beanspruchung des Gewebes sogar zusätzliche Läsionen des Gewebes wie Blasen oder Hautrisse entstehen.

Eine Wundauflage gemäß EP 3 085 344 B1 umfasst eine Vliesstoffschicht, welche diskrete Gruppen von geradlinigen Einschnitten aufweist, wobei jede Gruppe von Einschnitten mindestens einen ersten und einen zweiten Einschnitt umfasst, welche sich von einem gemeinsamen Startpunkt aus zu voneinander beabstandeten Endpunkten erstrecken. Gemäß EP 3 085 344 B1 wird eine Wundauflage durch das Vorhandensein von diskreten Gruppen von Einschnitten flexibler, so dass sich die Wundauflage besser an den Körper des Anwenders anpassen kann und die Wundauflage unter einer geringeren mechanischen Belastung steht. Die Einschnitte einer Gruppe von Einschnitten sind gemäß EP 3 085 344 B1 beispielsweise V-förmig oder in Form eines Sternes angeordnet. An den gemeinsamen Startpunkten eines ersten und zweiten Einschnitts einer Gruppe von geradlinigen Einschnitten kommt es jedoch, insbesondere wenn der erste und zweite Einschnitt nicht kollinear angeordnet sind, zu einer Ablösung von Fasern und Teilen der Vliesstoffschicht aus dem Vlies. Durch eine Ablösung von Fasern und Teilen der Vliesstoffschicht, insbesondere an dem gemeinsamen Startpunkt des ersten und zweiten Einschnitts, kann es zu einer Einschränkung der Funktionalität der Wundauflage kommen. Bei einem Einsatz der bekannten Wundauflage kommt es daher in vielen Fällen noch zu einer Beeinträchtigung der Wundheilung. Ferner hat sich gezeigt, dass die aus dem Vlies abgelösten Fasern und Teile der Vliesstoffschicht in die Wunde eindringen können. Entsprechende Vliesstoffrückstände in der Wunde können die Wundheilung stören. Der Wundheilungsprozess ist daher in vielen Fällen trotz Spannungsentlastung der Wundauflage beeinträchtigt.

Ein Wundpflegeartikel gemäß DE 10 2011 002 268 A1 umfasst ein Vlies- oder Airlaidmaterial, welches ein Muster aus Inzisionen und/oder Stanzungen aufweist, um den Flüssigkeitseintritt in den Wundpflegeartikel zu steigern. Gemäß DE 10 2011 002 268 A1 ist der Flüssigkeitseintritt in den Wundpflegeartikel durch entsprechende Inzisionen und/oder Stanzungen erhöht. Es hat sich herausgestellt, dass der erhöhte Flüssigkeitseintritt und die entsprechenden Inzisionen bzw. Stanzungen jedoch dazu führen können, dass sich der Wundpflegeartikel von der Wunde ablöst und die Tragedauer des Wundpflegeartikels verkürzt ist. Ferner hat sich herausgestellt, dass bei einem Einsatz des Wundpflegeartikels an Körperstellen, an denen eine hohe Scherspannung auf den Wundpflegeartikel einwirkt, z. B. durch Beugen eines Knies oder eines Ellbogens, die Funktionalität des Wundpflegeartikels durch eine teilweise Ablösung von der Wunde eingeschränkt sein kann. Darüber hinaus wurde in vielen Fällen beobachtet, dass es durch entsprechende Inzisionen und/oder Stanzungen, welche Material aus dem Vliesmaterial entfernen, zu einer Desintegration des Wundpflegeartikels kommt, wodurch die Absorptionsfähigkeit und Funktionalität des Wundpflegeartikels vermindert ist. Trotz eines erhöhten Flüssigkeitseintritts in das Absorptionsmaterial des Wundpflegeartikels kann der Wundheilungsprozess daher beeinträchtigt und der Tragekomfort für den Anwender vermindert sein.

CN213465619U offenbart einen Wundverband mit erhöhtem Tragekomfort der folgende Schichten umfasst: eine Trägerschicht, eine unter der Trägerschicht angeordnete absorbierende Schicht, eine unter der absorbierenden Schicht angeordnete Kontaktschicht und und eine auf der Kontaktschicht angeordnete untere Schutzschicht. Die untere Schutzschicht und die absorbierende Schicht sind mit mindestens einer Inzisionsgruppe versehen. Die Inzisionsgruppe besteht aus einer S-förmigen Inzision; der Bogenbereich der S-förmigen Inzision beträgt 30° bis 150° und der Radius 2 mm bis 8 mm.

Angesichts dieser Probleme im Stand der Technik liegt der Erfindung die Aufgabe zugrunde, eine Wundauflage bereitzustellen, welche eine verbesserte Wundheilung ermöglicht.

Erfindungsgemäß wird diese Aufgabe durch die im Patentanspruch 1 angegebene Weiterbildung der bekannten Wundauflagen gelöst.

Diese Erfindung geht auf die Erkenntnis zurück, dass die Wundheilung mit den bekannten Wundauflagen trotz einer Spannungsentlastung durch Einschnitte oder Stanzungen unzureichend ist. Es hat sich gezeigt, dass bogenförmige Einschnitte mit einem Krümmungsradius in einem Bereich von 0,5 mm bis 20 mm, und einer an den bogenförmigen Einschnitt angelegten Tangente, welche einen Winkel von 30° bis 150°, bevorzugt 45° bis 135°, bevorzugter 60° bis 120° zu der Maschinenrichtung des Vlieses aufweist, bessere Wundheilungsergebnisse erzielen. Überraschenderweise hat sich gezeigt, dass mittels bogenförmiger Einschnitte besonders viele Fasern in Maschinenrichtung eines Vlieses geschnitten werden können, wodurch eine hervorragende Spannungsentlastung und eine Dehnbarkeit des Vlieses herbeigeführt werden können. Darüber hinaus bleibt die Absorptionsfähigkeit der Wundauflage mittels bogenförmiger Einschnitte erhalten und Vliesstoffrückstände in der Wunde werden vermieden. Der Wundheilungsprozess wird mit einer erfindungsgemäßen Wundauflage somit optimal gefördert. Einschnitte sind im Kontext der vorliegenden Erfindung Einschnitte in ein Material, beispielsweise in das Vlies, ohne dass Material entnommen wird, weil die Einschnitte im Gegensatz zu Ausstanzungen keinen Bereich des Vlieses vollständig umlaufen. Gegenüber Ausstanzungen, bei denen Material entnommen wird, haben Einschnitte den Vorteil, dass es nicht zu einer Desintegration des Materials, beispielsweise des Vlieses, kommt. Ausstanzungen, bei denen Vliesmaterial entnommen wird, wie beispielsweise kreisförmige Ausstanzungen, haben den Nachteil, dass es zu einer Desintegration des Vlieses und so zu einer verminderten Absorptionsfähigkeit entsprechender Wundauflagen kommen kann. Es hat sich herausgestellt, dass Einschnitte, bei denen im Wesentlichen kein Material aus dem Vlies entfernt wird, für die Wundversorgung besonders zweckmäßig sind, da das Vlies intakt bleibt und so eine gute Absorptionsfähigkeit gewährleistet wird. Bevorzugt beträgt ein maximaler Abstand zwischen den entlang der längsten Erstreckung des bogenförmigen Einschnitts verlaufenden gegenüberliegenden Seiten des bogenförmigen Einschnitts, bzw. die Breite des Einschnitts, ≤ 1 mm. Dieser maximale Abstand bezieht sich insbesondere auf einen Zustand, bei dem die Wundauflage nicht an die Wunde angebracht ist.

Erfindungsgemäß ist es zur Verbesserung der Dehnbarkeit des Vlieses in Maschinenrichtung vorgesehen, dass der mindestens eine bogenförmige Einschnitt des Vlieses, bevorzugt jeder bogenförmige Einschnitt einer Vielzahl von bogenförmigen Einschnitten des Vlieses, einen Krümmungsradius in einem Bereich von 3 mm bis 10 mm aufweist. Eine stärkere Krümmung der bogenförmigen Einschnitte hat den Vorteil, dass die bogenförmigen Einschnitte so auf der Wundauflage angeordnet werden können, dass benachbarte bogenförmige Einschnitte einen möglichst geringen Abstand voneinander haben. Eine Anordnung von benachbarten bogenförmigen Einschnitten mit einem geringen Abstand ermöglicht, dass viele der in Maschinenrichtung verlaufenden Fasern durch die benachbarten bogenförmigen Einschnitte durchtrennt werden, wodurch eine erhöhte Nachgiebigkeit in Maschinenrichtung gewährleistet wird. Wenn das Vlies mehr als einen bogenförmigen Einschnitt umfasst, so sind die bogenförmigen Einschnitte des Vlieses voneinander beabstandet.

Bei einer bevorzugten Ausführungsform der Erfindung ist die Wundauflage dadurch gekennzeichnet, dass der mindestens eine bogenförmige Einschnitt des Vlieses eine Länge in einem Bereich von 1 mm bis 15 mm, bevorzugt 2 mm bis 10 mm aufweist. Bevorzugt weisen alle bogenförmigen Einschnitte des Vlieses eine Länge in einem Bereich von 1 mm bis 15 mm, bevorzugt 2 mm bis 10 mm auf. Eine solche Länge des mindestens einen bogenförmigen Einschnitts hat den Vorteil, dass durch die Durchtrennung der Fasern entlang der Länge des bogenförmigen Einschnitts die Dehnbarkeit der Wundauflage in der Maschinenrichtung des Vlieses erhöht ist und die Wundauflage dennoch eine hohe strukturelle Integrität aufweist. Vorzugsweise weist das Vlies eine Vielzahl von bogenförmigen Einschnitten mit einer Länge in einem Bereich von 1 mm bis 15 mm, bevorzugt 2 mm bis 10 mm auf.

Es hat sich im Hinblick auf die strukturelle Integrität des Vlieses und die Vermeidung von Unstetigkeitsstellen als zweckmäßig erwiesen, dass eine Länge zwischen einem ersten Endpunkt des bogenförmigen Einschnitts und einem zweiten Endpunkt des bogenförmigen Einschnitts in einem Bereich von 0,5 mm bis 12 mm, bevorzugt 1 mm bis 9 mm ausgeführt ist. Es hat sich gezeigt, dass durch die Vermeidung von Unstetigkeitsstellen eine die Wundheilung beeinträchtigende Ablösung von Fasern oder von Teilen der Vliesstoffschicht vermieden werden kann.

Eine durch den ersten Endpunkt des bogenförmigen Einschnitts und den zweiten Endpunkt des bogenförmigen Einschnitts verlaufende Gerade kann einen Winkel von 30° bis 150°, bevorzugt 45° bis 135°, bevorzugter 60° bis 120° zu der Maschinenrichtung des Vlieses aufweisen. Dies hat den Vorteil, dass der bogenförmige Einschnitt eine Vielzahl der Fasern des Vlieses orthogonal zur Faserausrichtung durchtrennt, wodurch die Dehnbarkeit des Vlieses in Maschinenrichtung verbessert wird.

Erfindungsgemäß ist im Sinne einer verbesserten Dehnbarkeit des Vlieses vorgesehen, dass der bogenförmige Einschnitt des Vlieses zweizählig drehsymmetrisch bezüglich einer senkrecht zu der flächigen Erstreckung der Wundauflage verlaufenden Achse ist. Bevorzugt weist das Vlies eine Vielzahl von derartigen zweizählig drehsymmetrischen bogenförmigen Einschnitten auf. Zweizählig drehsymmetrische bogenförmige Einschnitte können beispielsweise S-förmig oder wellenförmig vorliegen. Wenn der bogenförmige Einschnitt zweizählig drehsymmetrisch ausgeführt ist, wird der bogenförmigen Einschnitte durch eine Verdrehung um 180° auf sich selbst abgebildet.

Wie vorstehend bereits angesprochen, kann der bogenförmige Einschnitt als S-förmiger Einschnitt oder wellenförmiger Einschnitt ausgeführt sein. Dabei hat es sich in Bezug auf die Herstellung der Wundauflage als zweckmäßig herausgestellt, wenn alle bogenförmigen Einschnitte einer Vielzahl von bogenförmigen Einschnitten eine identische Form aufweisen. Die bogenförmigen Einschnitte der Vielzahl von bogenförmigen Einschnitten können eine unterschiedliche räumliche Ausrichtung entlang der flächigen Erstreckung des Vlieses, insbesondere entlang der Maschinenrichtung und/oder der zur Maschinenrichtung orthogonalen Richtung, aufweisen und durch Symmetrieoperationen, beispielsweise Spiegelung, Parallelverschiebung und/oder Drehung ineinander überführbar sein. Beispielsweise können die einzelnen bogenförmigen Einschnitte einer Vielzahl von bogenförmigen Einschnitten die gleiche Form aufweisen, jedoch gespiegelt, parallel verschoben und/oder gedreht zueinander vorliegen.

Bei einer besonders bevorzugten Ausführungsform der Erfindung weist das Vlies eine Vielzahl von bogenförmigen Einschnitten auf, die voneinander beabstandet sind. Durch das Vorhandensein einer Vielzahl von bogenförmigen Einschnitten in dem Vlies werden viele Fasern in Maschinenrichtung durchtrennt und die Dehnbarkeit des Vlieses in Maschinenrichtung wird erhöht, so dass sich die Wundauflage besser an den Körper des Anwenders anpassen kann. Überraschenderweise hat sich herausgestellt, dass die Funktionalität einer erfindungsgemäßen Wundauflage mit einer Vielzahl von bogenförmigen Einschnitten sogar bei einem Einsatz an Körperstellen, an denen eine hohe Scherspannung auf die Wundauflage einwirkt, gewährleistet ist, da die bogenförmigen Einschnitte eine optimale Anpassung an entsprechende Körperstellen ermöglichen. Vorteilhafterweise erhöhen die bogenförmigen Einschnitte der erfindungsgemäßen Wundauflage den Tragekomfort für den Anwender durch eine Reduktion der mechanischen Beanspruchung der Wunde unter gleichzeitiger Vermeidung von Vliesstoff-Rückständen in der Wunde.

Im Sinne einer besonders vorteilhaften Dehnbarkeit des Vlieses in Maschinenrichtung hat es sich als günstig erwiesen, wenn mindestens zwei voneinander beabstandete bogenförmige Einschnitte der Vielzahl von bogenförmigen Einschnitten einen Abstand in einem Bereich von 0,5 mm bis 10 mm, bevorzugt 1 mm bis 5 mm aufweisen. Ein solcher Abstand bezieht sich auf die minimale Entfernung zwischen zwei auf den jeweiligen zwei voneinander beabstandeten bogenförmigen Einschnitten liegenden Punkten, d.h. zwischen einem ersten auf einem ersten bogenförmigen Einschnitt liegenden Punkt und einem zweiten auf einem von dem ersten bogenförmigen Einschnitt beabstandeten zweiten Einschnitt liegenden Punkt. Durch eine entsprechende Beabstandung von benachbarten bogenförmigen Einschnitten wird ein hoher Anteil der Fasern des Vlieses, insbesondere ein hoher Anteil der in der Maschinenrichtung des Vlieses ausgerichteten Fasern, durchtrennt, so dass die Wundauflage eine hervorragende Dehnbarkeit in Maschinenrichtung aufweist und Unstetigkeitsstellen vermieden werden. Bevorzugt weist die Mehrzahl der bogenförmigen Einschnitte der Vielzahl von bogenförmigen Einschnitten des Vlieses, besonders bevorzugt weisen alle bogenförmige Einschnitte der Vielzahl von bogenförmigen Einschnitten des Vlieses, zu ihren jeweiligen benachbarten bogenförmigen Einschnitten einen Abstand in einem Bereich von 0,5 mm bis 10 mm, bevorzugt 1 mm bis 5 mm auf.

Es hat sich in Bezug auf die Dehnbarkeit und die Vermeidung von Unstetigkeitsstellen als zweckmäßig erwiesen, wenn mindestens zwei voneinander beabstandete bogenförmige Einschnitte der Vielzahl von bogenförmigen Einschnitten entlang der Maschinenrichtung des Vlieses einen Abstand in einem Bereich von 0,5 mm bis 10 mm, bevorzugt 1 mm bis 5 mm aufweisen. Ein solcher Abstand zwischen zwei benachbarten bogenförmigen Einschnitten entlang der Maschinenrichtung des Vlieses ermöglicht eine besonders vorteilhafte Dehnbarkeit der Wundauflage, da die Mehrzahl der Fasern, welche in Maschinenrichtung des Vlieses angeordnet sind und die Dehnbarkeit des Vlieses in Maschinenrichtung mindern, durchtrennt vorliegen. Ein solcher Abstand entlang der Maschinenrichtung bezieht sich auf die minimale Entfernung zwischen zwei auf den jeweiligen zwei voneinander beabstandeten bogenförmigen Einschnitten liegenden Punkten entlang der Maschinenrichtung des Vlieses. Bevorzugt weist die Mehrzahl der bogenförmigen Einschnitte der Vielzahl von bogenförmigen Einschnitten des Vlieses, besonders bevorzugt weisen alle bogenförmige Einschnitte der Vielzahl von bogenförmigen Einschnitten des Vlieses, zu ihren jeweiligen benachbarten bogenförmigen Einschnitten entlang der Maschinenrichtung des Vlieses einen Abstand in einem Bereich von 0,5 mm bis 10 mm, bevorzugt 1 mm bis 5 mm auf. Benachbarte bogenförmige Einschnitte sind solche Einschnitte, welche in Maschinenrichtung oder quer zu der Maschinenrichtung, d.h. in der zur Maschinenrichtung orthogonalen Richtung, des Vlieses zueinander benachbart, insbesondere zueinander ohne dazwischen angeordnete Einschnitte direkt benachbart, sind.

Zusätzlich oder alternativ können mindestens zwei voneinander beabstandete bogenförmige Einschnitte der Vielzahl von bogenförmigen Einschnitten des Vlieses entlang einer zur Maschinenrichtung orthogonalen Richtung einen Abstand in einem Bereich von 0,5 mm bis 10 mm, bevorzugt 1 mm bis 5 mm aufweisen. Dies ermöglicht eine besonders gute Dehnbarkeit des Vlieses entlang einer Richtung der flächigen Erstreckung der Wundauflage, die orthogonal zu der Maschinenrichtung des Vlieses verläuft. Ein solcher Abstand entlang einer zur Maschinenrichtung orthogonalen Richtung bezieht sich auf die minimale Entfernung zwischen zwei auf den jeweiligen zwei voneinander beabstandeten bogenförmigen Einschnitten liegenden Punkten entlang der orthogonalen Richtung, d.h. entlang der flächigen Erstreckung der Wundauflage in orthogonaler Richtung zu der Maschinenrichtung des Vlieses. Bevorzugt weist die Mehrzahl der bogenförmigen Einschnitte der Vielzahl von bogenförmigen Einschnitten des Vlieses, besonders bevorzugt weisen alle bogenförmige Einschnitte der Vielzahl von bogenförmigen Einschnitten des Vlieses, zu ihren jeweiligen benachbarten bogenförmigen Einschnitten entlang der zur Maschinenrichtung orthogonalen Richtung einen Abstand in einem Bereich von 0,5 mm bis 10 mm, bevorzugt 1 mm bis 5 mm auf.

Eine erfindungsgemäße Wundauflage ist dann besonders dehnbar, wenn die bogenförmigen Einschnitte der Vielzahl von bogenförmigen Einschnitten des Vlieses zu ihren jeweiligen in Maschinenrichtung benachbarten Einschnitten entlang der Maschinenrichtung des Vlieses einen Abstand in einem Bereich von 0,5 mm bis 10 mm, bevorzugt 1 mm bis 5 mm aufweisen, und zu ihren jeweiligen in orthogonaler Richtung zu der Maschinenrichtung des Vlieses benachbarten Einschnitten entlang der orthogonalen Richtung einen Abstand in einem Bereich von 0,5 mm bis 10 mm, bevorzugt 1 mm bis 5 mm aufweisen.

Bei einer erfindungsgemäßen Wundauflage können die bogenförmigen Einschnitte so ausgeführt sein, dass mindestens zwei voneinander beabstandete bogenförmige Einschnitte der Vielzahl von bogenförmigen Einschnitten entlang der Maschinenrichtung eine Überlappung in einer Länge in einem Bereich von 0,1 mm bis 5 mm, bevorzugt 0,2 mm bis 2 mm aufweisen. Eine solche Überlappung von Einschnitten entlang der Maschinenrichtung hat zur Folge, dass die Mehrzahl der in Maschinenrichtung ausgerichteten Fasern, bevorzugt alle der in Maschinenrichtung ausgerichteten Fasern, zumindest einmal durchtrennt sind, wodurch die Dehnbarkeit der Wundauflage erhöht wird.

Die bogenförmigen Einschnitte einer erfindungsgemäßen Wundauflage können darüber hinaus so ausgeführt sein, dass mindestens zwei voneinander beabstandete bogenförmige Einschnitte der Vielzahl von bogenförmigen Einschnitten entlang der zur Maschinenrichtung orthogonalen Richtung eine Überlappung in einer Länge in einem Bereich von 0,1 mm bis 5 mm, bevorzugt 0,2 mm bis 2 mm aufweisen. Eine solche Überlappung von Einschnitten entlang der zur Maschinenrichtung orthogonalen Richtung hat zur Folge, dass die Mehrzahl der in Maschinenrichtung ausgerichteten Fasern, bevorzugt alle der in Maschinenrichtung ausgerichteten Fasern, zumindest einmal in der zur Maschinenrichtung orthogonalen Richtung durchtrennt sind, wodurch die Dehnbarkeit der Wundauflage erhöht wird. Eine besonders hohe Dehnbarkeit der Wundauflage kann erzielt werden, wenn die bogenförmigen Einschnitte so ausgeführt werden, dass mindestens zwei voneinander beabstandete bogenförmige Einschnitte in Maschinenrichtung eine Überlappung aufweisen und mindestens zwei voneinander beabstandete bogenförmige Einschnitte in der zur Maschinenrichtung orthogonalen Richtung eine Überlappung aufweisen. Die Wundauflage kann beispielsweise so ausgeführt werden, dass die Wundauflage ein Muster aus einer Vielzahl von voneinander beabstandeten Einschnitten aufweist, wobei mindestens zwei jeweils benachbarte Einschnitte des Musters entlang der Maschinenrichtung eine Überlappung aufweisen und mindestens zwei jeweils benachbarte Einschnitte des Musters entlang der zur Maschinenrichtung orthogonalen Richtung eine Überlappung aufweisen. Um eine besonders hohe Dehnbarkeit der Wundauflage zu erzielen, kann ein solches Muster beispielsweise so ausgeführt werden, dass eine Vielzahl von Einschnitten, bevorzugt jeder Einschnitt einer Vielzahl von Einschnitten, mit mindestens einem benachbarten Einschnitt eine Überlappung in Maschinenrichtung und/oder eine Überlappung in der zur Maschinenrichtung orthogonalen Richtung aufweist.

Wie vorstehend bereits erläutert, können mehrere bogenförmige Einschnitte der Vielzahl von bogenförmigen Einschnitten des Vlieses eine identische Form aufweisen. Bevorzugt weisen mindestens zwei voneinander beabstandete bogenförmige Einschnitte der Vielzahl von bogenförmigen Einschnitten, bevorzugt alle voneinander beabstandeten bogenförmigen Einschnitte der Vielzahl von bogenförmigen Einschnitten, eine identische Form auf, beispielsweise eine S-Form oder Wellenform. Diese können so angeordnet sein, dass die beabstandeten bogenförmigen Einschnitte ein Muster aus Einschnitten in dem Vlies bilden. In einer bevorzugten Ausführungsform weist die Wundauflage eine Vielzahl von Einschnitten auf, welche eine identische Form aufweisen, wobei entlang der Maschinenrichtung jeweils benachbarte Einschnitte entlang der Maschinenrichtung des Vlieses einen Abstand in einem Bereich von 0,5 mm bis 10 mm, bevorzugt 1 mm bis 5 mm aufweisen, und wobei entlang der orthogonal zu der Maschinenrichtung verlaufenden Richtung der flächigen Erstreckung der Wundauflage jeweils benachbarte Einschnitte entlang der orthogonalen Richtung einen Abstand in einem Bereich von 0,5 mm bis 10 mm, bevorzugt 1 mm bis 5 mm aufweisen.

Bei einer erfindungsgemäßen Wundauflage kann das Vlies einen Randbereich und einen zentralen Bereich aufweisen, wobei der Randbereich und/oder der zentrale Bereich die Vielzahl von bogenförmigen Einschnitten aufweist. Je nach Anwendung hat es sich als besonders vorteilhaft herausgestellt, wenn nur der Randbereich, nur der zentrale Bereich, oder sowohl der Randbereich als auch der zentrale Bereich bogenförmige Einschnitte aufweisen. Für eine Anwendung an Körperstellen, an denen eine hohe Scherspannung auf die Wundauflage einwirkt, z. B. Knie oder Ellbogen, kann es für den Tragekomfort des Anwenders besonders günstig sein, wenn sich die Einschnitte sowohl im Randbereich als auch im zentralen Bereich befinden. So kann der Anwender das Knie bzw. den Ellenbogen beugen ohne dass es zu einer unangenehmen mechanischen Beanspruchung der Wunde oder einer Ablösung der Wundauflage kommt. Bei anderen Anwendungen, beispielsweise kleineren Körperstellen, an denen unterschiedliche mechanische Belastungen auftreten, beispielsweise Zehen, Fersen und Fingern, kann es vorteilhaft sein, wenn nur der Randbereich oder nur der zentrale Bereich die bogenförmige Einschnitte aufweist, beispielsweise um eine unterschiedliche mechanische Belastung an Übergängen zwischen Körperstellen, wie an einem Übergang zwischen Zehen und Fußsohle, auszugleichen.

In einer bevorzugten Ausführungsform sind die bogenförmigen Einschnitte der Vielzahl von bogenförmigen Einschnitten gleichmäßig über das Vlies verteilt. Bei einem Vlies mit gleichmäßig verteilten Einschnitten sind mindestens 25%, bevorzugt 50%, bevorzugter 75% der Einschnitte der Vielzahl von bogenförmigen Einschnitten so ausgestaltet, dass ein Abstand zwischen jeweils benachbarten Einschnitten in etwa gleich ist und/oder dass Einschnitte durch Symmetrieoperationen ineinander überführbar sind. Eine Symmetrieoperation kann eine Spiegelung, eine Parallelverschiebung und/oder eine Drehung umfassen. Beispielsweise weisen eine Mehrzahl der Einschnitte der gleichmäßig verteilten Einschnitte zu ihren jeweils benachbarten Einschnitten entlang der Maschinenrichtung und/oder entlang der zur Maschinenrichtung orthogonalen Richtung einen in etwa gleichen Abstand auf, z. B. einen Abstand in einem Bereich von 0,5 mm bis 10 mm, bevorzugt 1 mm bis 5 mm. Das Vlies kann so ausgeführt sein, dass eine solche gleichmäßige Verteilung von bogenförmigen Einschnitten im Randbereich, im zentralen Bereich, oder über das gesamte Vlies sowohl im Randbereich als auch im zentralen Bereich, vorliegt. Die bogenförmigen Einschnitte können beispielsweise gleichmäßig über den Randbereich und den zentralen Bereich verteilt sein. Eine gleichmäßige Verteilung der bogenförmige Einschnitte ermöglicht eine gleichmäßige Dehnbarkeit der Wundauflage, wodurch die Wundauflage für Körperstellen, an denen eine hohe Scherspannung auf die Wundauflage einwirkt, besonders geeignet ist. Die erfindungsgemäße Wundauflage umfasst das Vlies und wahlweise weitere Schichten und/oder Materialien, beispielsweise eine Klebefolie. In einer Ausführungsform besteht die Wundauflage aus dem Fasern umfassenden Vlies.

Bei einer bevorzugten Ausführungsform umfasst das Vlies ein superabsorbierendes Polymer. Ein Vlies mit einem superabsorbierenden Polymer hat den Vorteil, dass durch das osmotische Potential des superabsorbierenden Polymers die Aufnahme von Wundflüssigkeit in die Wundauflage gesteigert wird. Superabsorbierende Polymere sind Kunststoffe, die in der Lage sind, ein Vielfaches ihres Eigengewichts, beispielsweise bis zu einem 1000-fachen des Eigengewichts, an Flüssigkeiten aufzusaugen. Superabsorbierende Polymere können beispielsweise Copolymere aus Acrylsäure und Natriumacrylat sein, wobei das Verhältnis der beiden Monomere zueinander variieren kann. Zusätzlich kann ein Kernvernetzer (Core-Cross-Linker, CXL) der Monomerlösung zugesetzt werden, der die gebildeten langkettigen Polymermoleküle stellenweise untereinander durch chemische Brücken verbindet. Durch diese Brücken wird das Polymer wasserunlöslich.

Bei einer bevorzugten Ausführungsform ist das superabsorbierende Polymer partikelförmig und/oder faserförmig ausgeführt. Ein partikelförmiges superabsorbierendes Polymer kann beispielsweise in Form eines Granulats und/oder eines Pulvers vorliegen. Es hat sich als zweckmäßig herausgestellt, wenn das partikelförmige superabsorbierende Polymer eine Partikelgröße in einem Bereich von 1 µm bis 1000 µm, bevorzugt 100 µm bis 1000 µm aufweist. Ein faserförmiges superabsorbierendes Polymer kann beispielsweise in Form einer oder mehrerer Fasern, eines Fasergewirkes, eines Fasergeleges, eines Faservlieses und/oder einer Faserwatte vorliegen. Die Partikelform, beispielsweise Granulat- oder Pulverform, eines superabsorbierenden Polymers hat sich sehr bewährt und ist daher besonders bevorzugt, da sie sich gut in ein Vlies einbringen lässt. Ebenso ist die Faserform eines superabsorbierenden Polymers besonders bevorzugt, da es sich hierbei um ein sowohl im trockenen als auch im gequollenen Zustand sehr weiches Produkt handelt, das modellierbar ist und das eine geringe Abrasivität aufweist.

Die Erfindung bezieht sich darüber hinaus auf einen Wundverband, der eine erfindungsgemäße Wundauflage umfasst. Ein erfindungsgemäßer Wundverband ist für einen Einsatz bei der Wundversorgung hergerichtet und ermöglicht eine einfache Anwendung durch den Anwender. Bevorzugt ist ein erfindungsgemäßer Wundverband ein steriler Wundverband.

Bei einer bevorzugten Ausführungsform umfasst der Wundverband eine Trägerschicht, eine superabsorbierende Schicht, eine Polyurethan umfassende Schicht und/oder eine Wundkontaktschicht. Die Trägerschicht begünstigt die Stabilität des Wundverbandes und schützt vor einem Eindringen von Substanzen wie Schmutz, Wasser und Bakterien von außen in den Wundverband. Einen besonders guten Schutz bietet die Trägerschicht, wenn die Trägerschicht wasserdicht ausgeführt ist. Die superabsorbierende Schicht steigert die Aufnahme von Wundflüssigkeit und wird besonders bevorzugt in Wundverbänden verwendet, die für mittel bis stark exsudierende Wunden hergerichtet werden. Die superabsorbierende Schicht kann das Vlies der Wundauflage sein, welches ein superabsorbierendes Polymer umfasst, und/oder kann eine zusätzliche Schicht des Wundverbandes sein, welche ein superabsorbierendes Material, z.B. ein superabsorbierendes Polymer, umfasst. Ein erfindungsgemäßer Wundverband kann beispielsweise eine superabsorbierende Schicht aufweisen, welche in Form des Vlieses der Wundauflage mit einem superabsorbierendem Polymer oder in Form einer von dem Vlies separaten superabsorbierenden Schicht ausgeführt ist, oder der Wundverband kann zwei superabsorbierende Schichten aufweisen, wobei eine erste superabsorbierende Schicht das Vlies der Wundauflage mit einem superabsorbierendem Polymer ist und eine zweite superabsorbierende Schicht eine zusätzliche superabsorbierende Schicht ist. Eine superabsorbierende Schicht kann beispielsweise ein Absorptionsmaterial, wie Zellstofffasern, umfassen, in das ein superabsorbierendes Polymer, beispielsweise ein Natriumacrylat-Acrylsäure-Polymerisat, eingebettet ist. Um eine hohe Saugkraft der superabsorbierenden Schicht(en) zu gewährleisten, kann eine superabsorbierende Schicht superabsorbierende Polymere mit einem Gewichtsanteil von ≥ 15 Gew.-% bis ≤ 99 Gew.-%, bezogen auf das Gesamtgewicht der superabsorbierenden Schicht, aufweisen. Eine superabsorbierende Schicht kann in einem Wundverband beispielsweise zwischen einer Trägerschicht und dem Vlies angeordnet werden. Die superabsorbierende Schicht kann bzw. die superabsorbierenden Schichten können ein superabsorbierendes Polymer umfassen, wodurch eine gute Exsudataufnahme gewährleistet wird. Herstellungstechnisch hat es sich als günstig erwiesen, wenn ein superabsorbierendes Polymer einer superabsorbierenden Schicht(en) partikelförmig und/oder faserförmig ausgeführt wird, da so eine hervorragende Einbettung des superabsorbierenden Materials in den Wundverband gewährleistet wird. Ein besonderer Vorteil von faserförmigen superabsorbierenden Polymeren ist, dass eine hohe strukturelle Integrität der superabsorbierenden Schicht(en) erzielt wird, bei der es nicht zu einer Herauslösung von superabsorbierendem Polymer aus der superabsorbierenden Schicht kommt. Die Polyurethan umfassende Schicht fördert ein hervorragendes Exsudatmanagment und bietet ein optimales Wundmilieu für die Wundheilung. Im Hinblick auf eine gute Ableitung von Flüssigkeit aus der Wunde und einer Verringerung des Mazerationsrisikos hat es sich als besonders günstig erwiesen, wenn die Polyurethan umfassende Schicht Polyurethanschaum umfasst. Ein erfindungsgemäßer Wundverband kann beispielsweise so hergerichtet sein, dass eine Polyurethan umfassende Schicht zwischen einer Wundkontaktschicht und dem Vlies der Wundauflage angeordnet ist. Die Wundkontaktschicht erhöht den Tragekomfort für den Anwender. Beispielsweise minimiert die Wundkontaktschicht den Schmerz des Patienten bei der Anwendung und reduziert das Trauma für die Wunden und umgebendes Gewebe beim Wundverbandswechsel. Es hat sich herausgestellt, dass eine Wundkontaktschicht, welche Silikon umfasst, im Hinblick auf eine Verringerung der Schmerzen des Patienten und des Traumas für das Gewebe beim Wundverbandswechsel besonders geeignet ist.

Im Rahmen der Erfindung ist auch daran gedacht, dass die superabsorbierende Schicht, die Polyurethan umfassende Schicht und/oder die Wundkontaktschicht mindestens einen bogenförmigen Einschnitt, bevorzugt eine Vielzahl von bogenförmigen Einschnitten, umfassen. Bevorzugt weist der mindestens eine bogenförmige Einschnitt der superabsorbierenden Schicht, der Polyurethan umfassenden Schicht und/oder der Wundkontaktschicht, sofern vorhanden, in etwa gleiche Eigenschaften wie der mindestens eine Einschnitt des Vlieses auf, beispielsweise hinsichtlich Krümmungsradius, Länge des Einschnitts, Ausrichtung entlang der Maschinenrichtung, Symmetrie, Form, Beabstandung und Verteilung. Es hat sich als zweckmäßig herausgestellt, wenn ein aus den bogenförmigen Einschnitten in der superabsorbierenden Schicht, der Polyurethan umfassenden Schicht und/oder der Wundkontaktschicht gebildetes Muster identisch zu dem aus den bogenförmigen Einschnitten in dem Vlies gebildeten Muster ist. Sofern mehr als eine Schicht von der superabsorbierenden Schicht, der Polyurethan umfassenden Schicht und der Wundkontaktschicht bogenförmige Einschnitte aufweist, weisen diese Schichten bevorzugt ein identisches Muster aus Einschnitten auf. Beispielweise kann ein solches Muster aus Einschnitten dadurch entstehen, dass mehrere Schichten des Wundverbands, beispielsweise eine superabsorbierende Schicht, das Vlies und eine Polyurethan umfassende Schicht, zunächst aufeinander angeordnet werden und anschließend der mindestens eine bogenförmige Einschnitt eingeschnitten wird, wodurch die aufeinander angeordneten Schichten jeweils den mindestens einen bogenförmigen Einschnitt aufweisen. Es hat sich herausgestellt, dass es im Hinblick auf die Dehnbarkeit des Wundverbandes besonders günstig ist, wenn nicht nur das Vlies mindestens einen bogenförmigen Einschnitt aufweist, sondern zusätzlich mindestens eine weitere Schicht, beispielsweise die superabsorbierende Schicht und/oder die Polyurethan umfassende Schicht, mindestens einen bogenförmigen Einschnitt aufweist. Gemäß einer Ausführungsform ist die Wundkontaktschicht, wenn die Wundkontaktschicht ein dehnbares Material, beispielsweise Silikon, aufweist, ohne bogenförmige Einschnitte ausgeführt.

Ein erfindungsgemäßer Wundverband kann darüber hinaus eine oder mehrere abziehbare Schutzfolien aufweisen, welche von der Wundkontaktschicht abziehbar sind, um die Anwendung des Wundverbandes zu erleichtern. Ferner kann die Form des Wundverbands so ausgestaltet werden, dass die Form an die anatomischen Anforderungen der Körperstelle, für den der Wundverband hergerichtet wird, angepasst ist. Dabei kann ein Wundverband beispielsweise in Form eines Quadrats, Rechtecks, Kreises oder Ovals ausgeführt sein. Für eine Wundversorgung schwieriger Wundlokalisationen, beispielsweise des Sakralbereichs, des Ellenbogens oder der Ferse, kann die Form des Wundverbands zielgerichtet für die entsprechende Körperstelle hergerichtet werden.

Die Erfindung bezieht sich auch auf ein Wundversorgungskit mit einem steril in einer Packung aufgenommenen erfindungsgemäßen Wundverband. Bevorzugt ist in und/oder an der Packung eine Anweisung zur Verwendung der Wundauflage bei der Behandlung von Wunden vorgesehen. Das erfindungsgemäße Wundversorgungskit ermöglicht ein einfaches und sicheres Anwenden des Wundverbands auf einer Wunde.

Wie der vorstehenden Erläuterung erfindungsgemäßer Wundauflagen und Wundverbände zu entnehmen ist, betrifft die Erfindung auch die Verwendung einer erfindungsgemäßen Wundauflage zur Herstellung eines erfindungsgemäßen Wundverbands. Dazu kann die Wundauflage als Wundverband hergerichtet werden, beispielsweise durch Zusammenfügen der Wundauflage mit weiteren Schichten, wie einer superabsorbierenden Schicht, einer Polyurethan umfassenden Schicht und/oder einer Wundkontaktschicht.

Die Erfindung ist auch auf eine erfindungsgemäße Wundauflage, einen erfindungsgemäßen Wundverband und/oder ein erfindungsgemäßes Wundversorgungskit zur Verwendung bei der Wundversorgung gerichtet. Es hat sich herausgestellt, dass eine hervorragende Wundversorgung gewährleistet werden kann, wenn das Vlies einer Wundauflage mit bogenförmigen Einschnitten ausgeführt wird, welche den Tragekomfort für den Patienten signifikant verbessern.

Nachstehend wird die Erfindung unter Bezugnahme auf die Zeichnung, auf die hinsichtlich aller erfindungswesentlichen und in der Beschreibung nicht weiter herausgestellten Einzelheiten ausdrücklich verwiesen wird, erläutert. In der Zeichnung zeigt:
- Fig. 1: eine Wundauflage (1) und einen Wundverband (9) gemäß einer ersten Ausführungsform der Erfindung,
- Fig. 2: eine Wundauflage (1) und einen Wundverband (9) gemäß einer zweiten Ausführungsform der Erfindung,
- Fig. 3: schematische Skizzen einer Vielzahl von bogenförmigen Einschnitten (3).

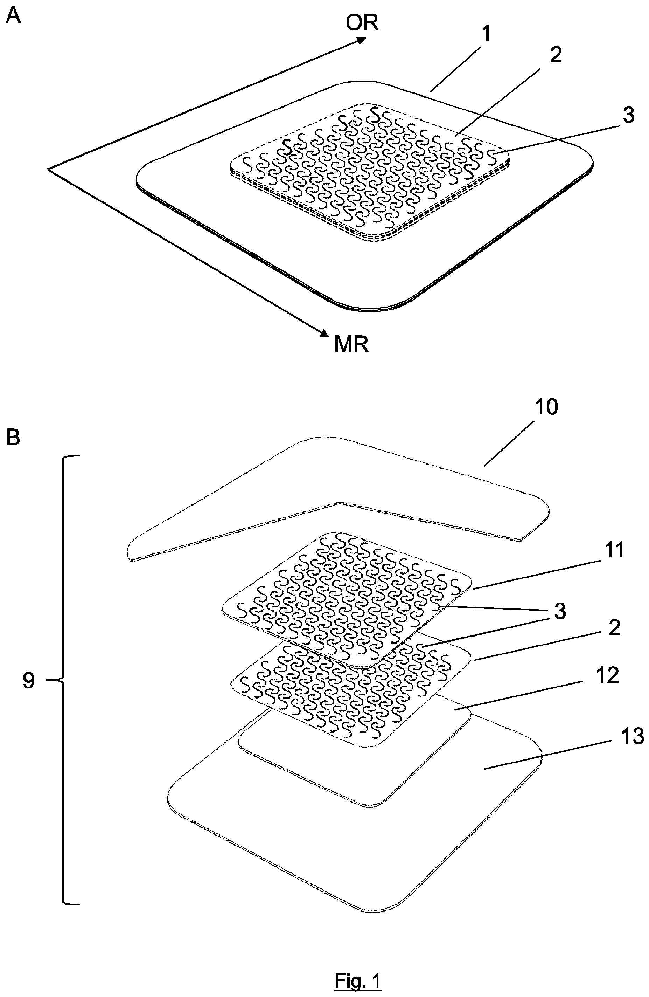

Fig. 1A zeigt eine Wundauflage (1) gemäß einer ersten Ausführungsform der Erfindung.

Die Wundauflage (1) umfasst ein Vlies (2) mit einer Vielzahl von bogenförmigen Einschnitten (3). Die Einschnitte (3) sind beispielhaft S-förmig ausgeführt. Die Einschnitte (3) der Vielzahl von bogenförmigen Einschnitten (3) sind gleichmäßig über das Vlies (2) verteilt. Die flächige Erstreckung der Wundauflage umfasst eine Maschinenrichtung (MR) und eine dazu orthogonale Richtung (OR). Die Fasern des Vlieses (2) weisen eine Vorzugsrichtung entlang der Maschinenrichtung (MR) auf, d.h. eine Mehrzahl der Fasern des Vlieses (2) sind entlang der Maschinenrichtung (MR) ausgerichtet. Durch die bogenförmigen Einschnitte (3) werden die Fasern, welche eine Dehnbarkeit entlang der Maschinenrichtung (MR) vermindern, durchtrennt, wodurch eine erfindungsgemäße Wundauflage (1) mit bogenförmigen Einschnitten (3) eine hervorragende Dehnbarkeit aufweist. Die Wundauflage (1) kann aus dem Vlies (2) bestehen oder weitere Materialien und/oder Schichten umfassen. Fig. 1B zeigt einen Wundverband (9), welcher das Vlies (2) umfasst und weiter eine Trägerschicht (10), eine superabsorbierende Schicht (11), eine Polyurethan umfassende Schicht (12) und eine Wundkontaktschicht (13) umfasst. Die superabsorbierende Schicht (11) ist zwischen der Trägerschicht (10) und dem Vlies (2) angeordnet. Die Wundauflage (1) besteht aus dem Vlies (2) und ist zwischen der superabsorbierenden Schicht (11) und der Polyurethan umfassenden Schicht (12) angeordnet. Die Polyurethan umfassende Schicht (12) ist zwischen dem Vlies (2) und der Wundkontaktschicht (13) angeordnet. Die superabsorbierende Schicht (11) umfasst eine Vielzahl von bogenförmigen Einschnitten (3). Bevorzugt ist ein durch die bogenförmige Einschnitte (3) der superabsorbierenden Schicht (11) gebildetes Muster identisch zu einem durch die bogenförmigen Einschnitte (3) des Vlieses (2) gebildeten Muster.

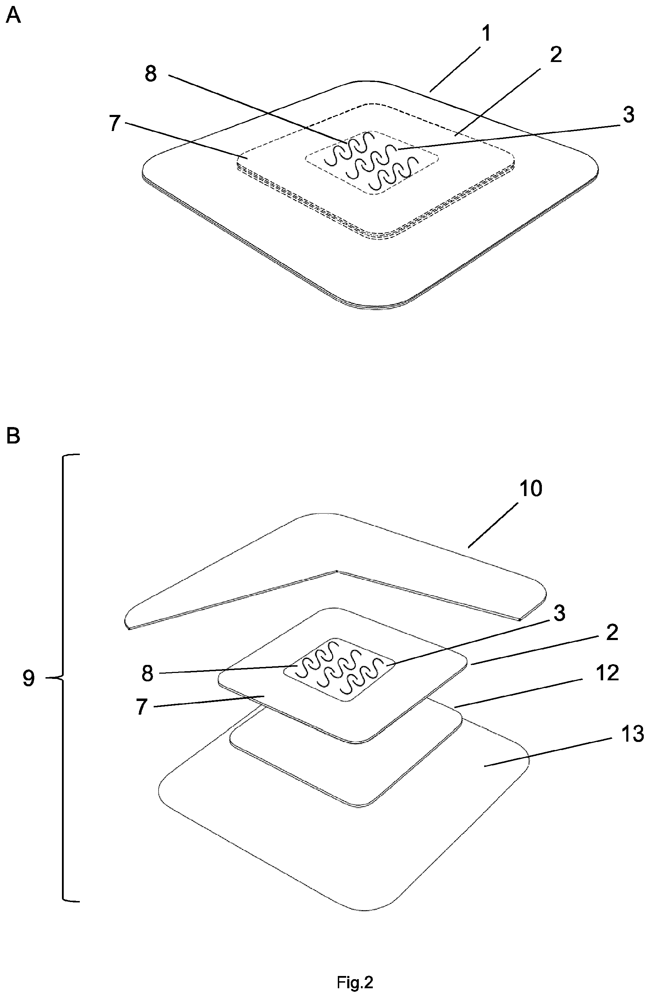

Fig. 2 zeigt eine Wundauflage (1) gemäß einer zweiten Ausführungsform der Erfindung.

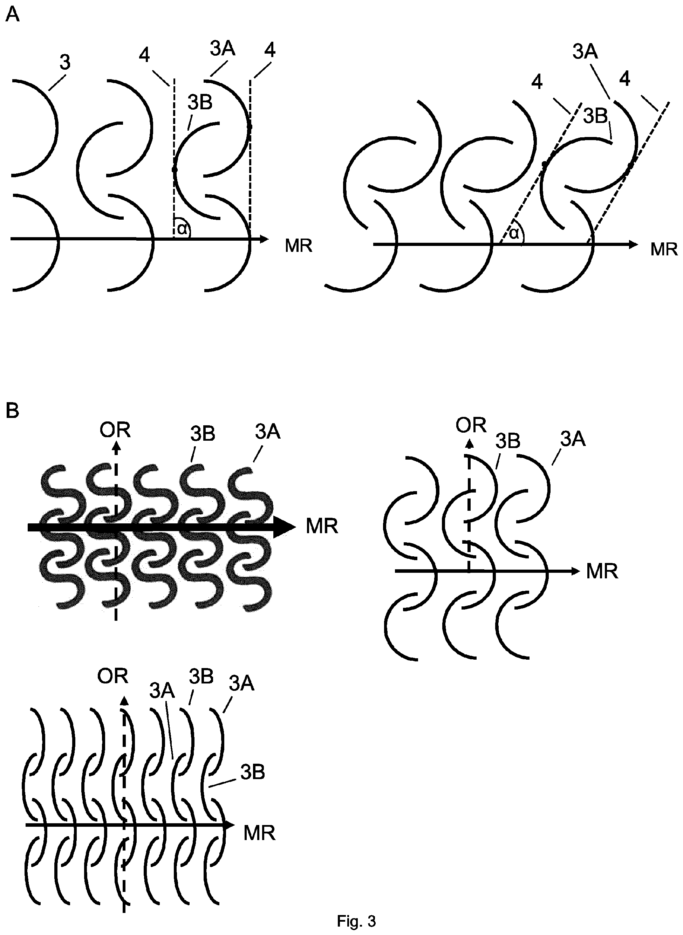

Die Wundauflage (1) umfasst ein Vlies (2) mit einer Vielzahl von bogenförmigen Einschnitten (3) in einem zentralen Bereich (8) des Vlieses (2). Die Einschnitte (3) sind beispielhaft S-förmig ausgeführt. Ein Randbereich (7) des Vlieses (2) weist hier beispielhaft keine bogenförmigen Einschnitte (3) auf. Die Einschnitte (3) der Vielzahl von bogenförmigen Einschnitten (3) sind gleichmäßig über den zentralen Bereich (8) des Vlieses (2) verteilt. Fig. 3B zeigt einen Wundverband (9), welcher ein Vlies (2) umfasst und weiter eine Trägerschicht (10), eine Polyurethan umfassende Schicht (12) und eine Wundkontaktschicht (13) umfasst. Die Wundauflage (1) besteht aus dem Vlies (2) und ist zwischen der Trägerschicht (10) und der Polyurethan umfassenden Schicht (12) angeordnet. Die Polyurethan umfassende Schicht (12) ist zwischen dem Vlies (2) und der Wundkontaktschicht (13) angeordnet.

Fig. 3 zeigt drei beispielhafte Muster von Einschnitten (3A, 3B), die durch eine Vielzahl von Einschnitten (3A, 3B), die voneinander entlang der Maschinenrichtung (MR) und entlang der zu der Maschinenrichtung (MR) orthogonalen Richtung (OR) beabstandet sind, gebildet werden. Das Vlies (2) sowie weitere Schichten eines Wundverbandes (9), beispielsweise eine Trägerschicht (10), eine superabsorbierende Schicht (11), eine Polyurethan umfassende Schicht (12) und/oder eine Wundkontaktschicht (13), können entsprechende Muster aufweisen.

### Bezugszeichenliste

- 1: Wundauflage
- 2: Vlies
- 3: Bogenförmiger Einschnitt
- 3A, 3B: Voneinander beabstandete bogenförmige Einschnitte
- 4: Tangente
- 5: Erster Endpunkt des bogenförmigen Einschnitts
- 6: Zweiter Endpunkt des bogenförmigen Einschnitts
- 7: Randbereich
- 8: Zentraler Bereich
- 9: Wundverband
- 10: Trägerschicht
- 11: Superabsorbierende Schicht
- 12: Polyurethan umfassende Schicht
- 13: Wundkontaktschicht
- MR: Maschinenrichtung
- OR: Zur Maschinenrichtung orthogonale Richtung
- L1: Länge des bogenförmigen Einschnitts
- L2: Länge zwischen einem ersten Endpunkt und einem zweiten Endpunkt des bogenförmigen Einschnitts
- L3: Länge einer Überlappung entlang der Maschinenrichtung
- L4: Länge eines Abstandes entlang der Maschinenrichtung
- L5: Länge eines Abstandes entlang der zur Maschinenrichtung orthogonalen Richtung

## Patentansprüche

1. Wundauflage (1) mit einem Fasern umfassenden Vlies (2), wobei eine Mehrzahl der Fasern in einer Maschinenrichtung (MR) des Vlieses (2) ausgerichtet sind, wobei das Vlies (2) mindestens einen Einschnitt (3) aufweist, **dadurch gekennzeichnet, dass** der Einschnitt (3) ein bogenförmiger Einschnitt (3) mit einem Krümmungsradius in einem Bereich von 3 mm bis 10 mm ist, und eine an den bogenförmigen Einschnitt (3) angelegte Tangente (4) einen Winkel von 30° bis 150°, bevorzugt 45° bis 135°, bevorzugter 60° bis 120° zu der Maschinenrichtung (MR) des Vlieses (2) aufweist, wobei der bogenförmige Einschnitt (3) zweizählig drehsymmetrisch bezüglich einer senkrecht zu der flächigen Erstreckung der Wundauflage (1) verlaufenden Achse ist, wobei der bogenförmige Einschnitt (3) als S-förmiger Einschnitt oder wellenförmiger Einschnitt ausgeführt ist.

2. Wundauflage (1) nach Anspruch 1, **dadurch gekennzeichnet, dass** der bogenförmige Einschnitt (3) eine Länge in einem Bereich von 1 mm bis 15 mm, bevorzugt 2 mm bis 10 mm aufweist.

3. Wundauflage (1) nach einem der vorhergehenden Ansprüche, **dadurch gekennzeichnet, dass** eine Länge zwischen einem ersten Endpunkt (5) des bogenförmigen Einschnitts (3) und einem zweiten Endpunkt (6) des bogenförmigen Einschnitts (3) in einem Bereich von 0,5 mm bis 12 mm, bevorzugt 1 mm bis 9 mm ausgeführt ist.

4. Wundauflage (1) nach einem der vorhergehenden Ansprüche, **dadurch gekennzeichnet, dass** eine durch den ersten Endpunkt (5) des bogenförmigen Einschnitts (3) und den zweiten Endpunkt (6) des bogenförmigen Einschnitts (3) verlaufende Gerade einen Winkel von 30° bis 150°, bevorzugt 45° bis 135°, bevorzugter 60° bis 120° zu der Maschinenrichtung (MR) des Vlieses (2) aufweist.

5. Wundauflage (1) nach einem der vorhergehenden Ansprüche, **dadurch gekennzeichnet, dass** das Vlies (2) eine Vielzahl von bogenförmigen Einschnitten (3) aufweist, die voneinander beabstandet sind.

6. Wundauflage (1) nach Anspruch 5, **dadurch gekennzeichnet, dass** mindestens zwei voneinander beabstandete, benachbarte bogenförmige Einschnitte (3A, 3B) der Vielzahl von bogenförmigen Einschnitten (3) einen Abstand in einem Bereich von 0,5 mm bis 10 mm, bevorzugt 1 mm bis 5 mm aufweisen.

7. Wundauflage (1) nach einem der Ansprüche 5-6, **dadurch gekennzeichnet, dass** mindestens zwei voneinander beabstandete, benachbarte bogenförmige Einschnitte (3A, 3B) der Vielzahl von bogenförmigen Einschnitten (3) entlang der Maschinenrichtung (MR) des Vlieses (2) einen Abstand in einem Bereich von 0,5 mm bis 10 mm, bevorzugt 1 mm bis 5 mm aufweisen.

8. Wundauflage (1) nach einem der Ansprüche 5-7, **dadurch gekennzeichnet, dass** mindestens zwei voneinander beabstandete, benachbarte bogenförmige Einschnitte (3A, 3B) der Vielzahl von bogenförmigen Einschnitten (3) entlang einer zur Maschinenrichtung (MR) orthogonalen Richtung (OR) einen Abstand in einem Bereich von 0,5 mm bis 10 mm, bevorzugt 1 mm bis 5 mm aufweisen.

9. Wundauflage (1) nach einem der Ansprüche 5-8, **dadurch gekennzeichnet, dass** mindestens zwei voneinander beabstandete bogenförmige Einschnitte der Vielzahl von bogenförmigen Einschnitten (3) entlang der Maschinenrichtung (MR) eine Überlappung in einer Länge in einem Bereich von 0,1 mm bis 5 mm, bevorzugt 0,2 mm bis 2 mm aufweisen.

10. Wundauflage (1) nach einem der Ansprüche 5-9, **dadurch gekennzeichnet, dass** mindestens zwei voneinander beabstandete bogenförmige Einschnitte (3A, 3B) der Vielzahl von bogenförmigen Einschnitten (3), bevorzugt alle voneinander beabstandeten bogenförmigen Einschnitte (3) der Vielzahl von bogenförmigen Einschnitten (3), eine identische Form aufweisen.

11. Wundauflage (1) nach einem der Ansprüche 5-10, **dadurch gekennzeichnet, dass** das Vlies (2) einen Randbereich (7) und einen zentralen Bereich (8) aufweist, wobei der Randbereich (7) und/oder der zentrale Bereich (8) die Vielzahl von bogenförmigen Einschnitten (3) aufweist.

12. Wundauflage (1) nach einem der Ansprüche 5-11, **dadurch gekennzeichnet, dass** die bogenförmigen Einschnitte (3) der Vielzahl von bogenförmigen Einschnitten (3) gleichmäßig über das Vlies (2) verteilt sind.

13. Wundauflage (1) nach einem der Ansprüche 5-12, **dadurch gekennzeichnet, dass** das Vlies (2) ein superabsorbierendes Polymer umfasst.

14. Wundauflage (1) nach Anspruch 13, **dadurch gekennzeichnet, dass** das superabsorbierende Polymer partikelförmig und/oder faserförmig ausgeführt ist.

15. Wundverband (9), umfassend eine Wundauflage (1) nach einem der Ansprüche 1-14.

16. Wundverband (9) nach Anspruch 15, **dadurch gekennzeichnet, dass** der Wundverband (9) eine Trägerschicht (10), eine superabsorbierende Schicht (11), eine Polyurethan umfassende Schicht (12) und/oder eine Wundkontaktschicht (13) umfasst.

17. Wundverband (9) nach Anspruch 16, **dadurch gekennzeichnet, dass** die Trägerschicht (10) wasserdicht ist.

18. Wundverband (9) nach einem der Ansprüche 16-17, **dadurch gekennzeichnet, dass** die superabsorbierende Schicht (11) ein superabsorbierendes Polymer umfasst.

19. Wundverband (9) nach einem der Ansprüche 16-18, **dadurch gekennzeichnet, dass** die Polyurethan umfassende Schicht (12) Polyurethanschaum umfasst.

20. Wundverband (9) nach einem der Ansprüche 16-19, **dadurch gekennzeichnet, dass** die Wundkontaktschicht (13) Silikon umfasst.

21. Wundverband (9) nach einem der Ansprüche 16-20, **dadurch gekennzeichnet, dass** die superabsorbierende Schicht (11), die Polyurethan umfassende Schicht (12) und/oder die Wundkontaktschicht (13) mindestens einen bogenförmigen Einschnitt (3), bevorzugt eine Vielzahl von bogenförmigen Einschnitten (3), umfassen.

22. Wundversorgungskit mit einem steril in einer Packung aufgenommenen Wundverband (9) nach einem der Ansprüche 15-21.

23. Wundversorgungskit nach Anspruch 22, **dadurch gekennzeichnet, dass** in und/oder an der Packung eine Anweisung zur Verwendung der Wundauflage (1) bei der Behandlung von Wunden vorgesehen ist.

24. Verwendung einer Wundauflage (1) nach einem der Ansprüche 1 bis 14 zur Herstellung eines Wundverbandes (9).

## Claims

1. Wound dressing (1) with a nonwoven (2) comprising fibres, wherein a plurality of the fibres are aligned in a machine direction (MR) of the nonwoven (2), wherein the nonwoven (2) has at least one incision (3), **characterized in that** the incision (3) is an arc-shaped incision (3) with a radius of curvature in a range from 3 mm to 10 mm, and a tangent (4) applied to the arc-shaped incision (3) has an angle of 30° to 150°, preferably 45° to 135°, more preferably 60° to 120°, to the machine direction (MR) of the nonwoven (2), wherein the arc-shaped incision (3) is doubly rotationally symmetrical with respect to an axis running perpendicularly to the planar extent of the wound dressing (1), wherein the arc-shaped incision (3) is designed as an S-shaped incision or undulating incision.

2. Wound dressing (1) according to Claim 1, **characterized in that** the arc-shaped incision (3) has a length in a range from 1 mm to 15 mm, preferably 2 mm to 10 mm.

3. Wound dressing (1) according to one of the preceding claims, **characterized in that** a length between a first end point (5) of the arc-shaped incision (3) and a second end point (6) of the arc-shaped incision (3) is designed in a range from 0.5 mm to 12 mm, preferably 1 mm to 9 mm.

4. Wound dressing (1) according to one of the preceding claims, **characterized in that** a straight line running through the first end point (5) of the arc-shaped incision (3) and the second end point (6) of the arc-shaped incision (3) has an angle of 30° to 150°, preferably 45° to 135°, more preferably 60° to 120°, to the machine direction (MR) of the nonwoven (2).

5. Wound dressing (1) according to one of the preceding claims, **characterized in that** the nonwoven (2) has a multiplicity of arc-shaped incisions (3) which are spaced apart from one another.

6. Wound dressing (1) according to Claim 5, **characterized in that** at least two adjacent arc-shaped incisions (3A, 3B), spaced apart from one another, of the multiplicity of arc-shaped incisions (3) have a spacing in a range from 0.5 mm to 10 mm, preferably 1 mm to 5 mm.

7. Wound dressing (1) according to one of Claims 5-6, **characterized in that** at least two adjacent arc-shaped incisions (3A, 3B), spaced apart from one another, of the multiplicity of arc-shaped incisions (3) have a spacing in a range from 0.5 mm to 10 mm, preferably 1 mm to 5 mm, along the machine direction (MR) of the nonwoven (2).

8. Wound dressing (1) according to one of Claims 5-7, **characterized in that** at least two adjacent arc-shaped incisions (3A, 3B), spaced apart from one another, of the multiplicity of arc-shaped incisions (3) have a spacing in a range from 0.5 mm to 10 mm, preferably 1 mm to 5 mm, along a direction (OR) orthogonal to the machine direction (MR).

9. Wound dressing (1) according to one of Claims 5-8, **characterized in that** at least two arc-shaped incisions, spaced apart from one another, of the multiplicity of arc-shaped incisions (3) have an overlap in a length in a range from 0.1 mm to 5 mm, preferably 0.2 mm to 2 mm, along the machine direction (MR).

10. Wound dressing (1) according to one of Claims 5-9, **characterized in that** at least two arc-shaped incisions (3A, 3B), spaced apart from one another, of the multiplicity of arc-shaped incisions (3), preferably all arc-shaped incisions (3), spaced apart from one another, of the multiplicity of arc-shaped incisions (3), have an identical shape.

11. Wound dressing (1) according to one of Claims 5-10, **characterized in that** the nonwoven (2) has an edge region (7) and a central region (8), wherein the edge region (7) and/or the central region (8) has the multiplicity of arc-shaped incisions (3).

12. Wound dressing (1) according to one of Claims 5-11, **characterized in that** the arc-shaped incisions (3) of the multiplicity of arc-shaped incisions (3) are distributed uniformly over the nonwoven (2).

13. Wound dressing (1) according to one of Claims 5-12, **characterized in that** the nonwoven (2) comprises a superabsorbent polymer.

14. Wound dressing (1) according to Claim 13, **characterized in that** the superabsorbent polymer is of particulate and/or fibrous design.

15. Wound dressing (9) comprising a wound dressing (1) according to one of Claims 1-14.

16. Wound dressing (9) according to Claim 15, **characterized in that** the wound dressing (9) comprises a carrier layer (10), a superabsorbent layer (11), a layer (12) comprising polyurethane and/or a wound contact layer (13).

17. Wound dressing (9) according to Claim 16, **characterized in that** the carrier layer (10) is waterproof.

18. Wound dressing (9) according to one of Claims 16-17, **characterized in that** the superabsorbent layer (11) comprises a superabsorbent polymer.

19. Wound dressing (9) according to one of Claims 16-18, **characterized in that** the layer (12) comprising polyurethane comprises polyurethane foam.

20. Wound dressing (9) according to one of Claims 16-19, **characterized in that** the wound contact layer (13) comprises silicone.

21. Wound dressing (9) according to one of Claims 16-20, **characterized in that** the superabsorbent layer (11), the layer (12) comprising polyurethane and/or the wound contact layer (13) comprise at least one arc-shaped incision (3), preferably a multiplicity of arc-shaped incisions (3).

22. Wound care kit comprising a wound dressing (9) according to one of Claims 15-21 accommodated in a sterile manner in a pack.

23. Wound care kit according to Claim 22, **characterized in that** instructions for using the wound dressing (1) in the treatment of wounds are provided in and/or on the pack.

24. Use of a wound dressing (1) according to one of Claims 1 to 14 for producing a wound dressing (9).

## Revendications

1. Compresse pour plaie (1) avec un voile (2) comprenant des fibres, une pluralité des fibres étant orientées dans une direction machine (MR) du voile (2), ledit voile (2) présentant au moins une incision (3), **caractérisée en ce que** l'incision (3) est une incision arquée (3) avec un rayon de courbure dans une plage de 3 mm à 10 mm, et une tangente (4) appliquée à l'incision arquée (3) présente un angle de 30° à 150°, de préférence de 45° à 135°, plus préférentiellement de 60° à 120° par rapport à la direction machine (MR) du voile (2), l'incision arquée (3) étant à symétrie de rotation d'ordre deux par rapport à un axe perpendiculaire à l'extension surfacique de la compresse pour plaie (1), l'incision arquée (3) étant réalisée comme une incision en forme de S ou une incision ondulée.

2. Compresse pour plaie (1) selon la revendication 1, **caractérisée en ce que** l'incision arquée (3) présente une longueur dans une plage de 1 mm à 15 mm, de préférence de 2 mm à mm.

3. Compresse pour plaie (1) selon l'une des revendications précédentes, **caractérisée en ce que** la longueur entre une première extrémité (5) de l'incision arquée (3) et une seconde extrémité (6) de l'incision arquée (3) est dans une plage de 0,5 mm à 12 mm, de préférence de 1 mm à 9 mm.

4. Compresse pour plaie (1) selon l'une des revendications précédentes, **caractérisée en ce qu'**une droite passant par la première extrémité (5) de l'incision arquée (3) et la seconde extrémité (6) de l'incision arquée (3) présente un angle de 30° à 150°, de préférence de 45° à 135°, plus préférentiellement de 60° à 120° par rapport à la direction machine (MR) du voile (2).

5. Compresse pour plaie (1) selon l'une des revendications précédentes, **caractérisée en ce que** le voile (2) présente une pluralité d'incisions arquées (3) qui sont espacés l'un de l'autre.

6. Compresse pour plaie (1) selon la revendication 5, **caractérisée en ce qu'**au moins deux incisions arquées adjacentes, espacés l'un de l'autre (3A, 3B) de la pluralité d'incisions arquées (3) présentent une distance dans une plage de 0,5 mm à 10 mm, de préférence de 1 mm à 5 mm.

7. Compresse pour plaie (1) selon l'une des revendications 5-6, **caractérisée en ce qu'**au moins deux incisions arquées adjacentes, espacés l'un de l'autre (3A, 3B) de la pluralité d'incisions arquées (3) le long de la direction machine (MR) du voile (2) présentent une distance dans une plage de 0,5 mm à 10 mm, de préférence de 1 mm à 5 mm.

8. Compresse pour plaie (1) selon l'une des revendications 5-7, **caractérisée en ce qu'**au moins deux incisions arquées adjacentes, espacés l'un de l'autre (3A, 3B) de la pluralité d'incisions arquées (3) le long d'une direction (OR) orthogonale à la direction machine (MR) présentent une distance dans une plage de 0,5 mm à 10 mm, de préférence de 1 mm à 5 mm.

9. Compresse pour plaie (1) selon l'une des revendications 5-8, **caractérisée en ce qu'**au moins deux incisions arquées espacés l'un de l'autre de la pluralité d'incisions arquées (3) le long de la direction machine (MR) présentent un chevauchement sur une longueur dans une plage de 0,1 mm à 5 mm, de préférence de 0,2 mm à 2 mm.

10. Compresse pour plaie (1) selon l'une des revendications 5-9, **caractérisée en ce qu'**au moins deux incisions arquées espacés l'un de l'autre (3A, 3B) de la pluralité d'incisions arquées (3), de préférence toutes les incisions arquées espacés l'un de l'autre (3) de la pluralité d'incisions arquées (3), présentent une forme identique.

11. Compresse pour plaie (1) selon l'une des revendications 5-10, **caractérisée en ce que** le voile (2) présente une zone périphérique (7) et une zone centrale (8), la zone périphérique (7) et/ou la zone centrale (8) présentant la pluralité d'incisions arquées (3).

12. Compresse pour plaie (1) selon l'une des revendications 5-11, **caractérisée en ce que** les incisions arquées (3) de la pluralité d'incisions arquées (3) sont répartis uniformément sur le voile (2).

13. Compresse pour plaie (1) selon l'une des revendications 5-12, **caractérisée en ce que** le voile (2) comprend un polymère superabsorbant.

14. Compresse pour plaie (1) selon la revendication 13, **caractérisée en ce que** le polymère superabsorbant est sous forme particulaire et/ou sous forme fibreuse.

15. Pansement (9), comprenant une compresse pour plaie (1) selon l'une des revendications 1-14.

16. Pansement (9) selon la revendication 15, **caractérisé en ce que** le pansement (9) comprend une couche de support (10), une couche superabsorbante (11), une couche comprenant du polyuréthane (12) et/ou une couche de contact avec la plaie (13).

17. Pansement (9) selon la revendication 16, **caractérisé en ce que** la couche de support (10) est étanche.

18. Pansement (9) selon l'une des revendications 16-17, **caractérisé en ce que** la couche superabsorbante (11) comprend un polymère superabsorbant.

19. Pansement (9) selon l'une des revendications 16-18, **caractérisé en ce que** la couche comprenant du polyuréthane (12) comprend une mousse de polyuréthane.

20. Pansement (9) selon l'une des revendications 16-19, **caractérisé en ce que** la couche de contact avec la plaie (13) comprend du silicone.

21. Pansement (9) selon l'une des revendications 16-20, **caractérisé en ce que** la couche superabsorbante (11), la couche comprenant du polyuréthane (12) et/ou la couche de contact avec la plaie (13) comprennent au moins une incision arquée (3), de préférence une pluralité d'incisions arquées (3).

22. Kit de traitement des plaies comprenant un pansement (9) stérile contenu dans un emballage selon l'une des revendications 15-21.

23. Kit de traitement des plaies selon la revendication 22, **caractérisé en ce qu'**une instruction d'utilisation de la compresse pour plaie (1) lors du traitement des plaies est prévue dans et/ou sur l'emballage.

24. Utilisation d'une compresse pour plaie (1) selon l'une des revendications 1 à 14 pour la fabrication d'un pansement (9).
